# EUROPEAN PATENT SPECIFICATION

(11) **EP 1 086 377 B1**
(45) Date of publication and mention of the grant of the patent: **06.02.2008**
(21) Application number: 99930078.3
(22) Date of filing: 07.06.1999
(51) Int. Cl.: G01N 33/569, C07K 14/35, C07K 14/01

(54) **A METHOD OF IDENTIFYING LIGANDS TO RNA POLYMERASE SIGMA 70 SUBUNIT**
VERFAHREN ZUM IDENTIFIZIEREN VON LIGANDEN DER RNA-POLYMERASE SIGMA 70 UNTEREINHEIT
PROCEDE D'IDENTIFICATION DE LIGANDS DE LA SOUS-UNITE SIGMA (70) DE L'ARN POLYMERASE

(30) Priority: 09.06.1998 IN MA123998; 17.07.1998 SE 9802573
(43) Date of publication of application: 28.03.2001
(73) Proprietor: AstraZeneca AB, 151 85 Södertälje (SE)
(72) Inventor: BALGANESH, Tanjore, AstraZeneca, R&D Bangalore, Bangalore 560 080 (IN); RAMACHANDRAN, Vasanthi, AstraZeneca, R&D Bangalore, Bangalore 560 080 (IN); SHARMA, Umender, AstraZeneca, R&D Bangalore, Bangalore 560 080 (IN)
(86) International application number: PCT/SE1999/000979
(87) International publication number: WO 1999/064866

(56) References cited:
- WO-A1-96/25170
- WO-A1-96/38478
- Database Medline, accession no. 93106978, Orsini G et al: "The Asia Gene of Bacteriophage T4 Codes for the Anti-Sigma 70 Protein"; & J. Bacteriol. 1993 Jan, 175(1), 85-93

### SEQUENCE LISTING

<110> ASTRA AKTIEBOLAG
<120> RNA Polymerase Assay
<130> R 1765
<140>
   <141>
<160> 8
<170> PatentIn Ver. 2.0
<210> 1
   <211> 90
   <212> PRT
   <213> Bacteriophage T4
<400> 1
   Met Asn Lys Asn Ile Asp Thr Val Arg Glu Ile Ile Thr Val Ala Ser
     1               5                  10                  15
   Ile Leu Ile Lys Phe Ser Arg Glu Asp Ile Val Glu Asn Arg Ala Asn
                20                  25                  30
   Phe Ile Ala Phe Leu Asn Glu Ile Gly Val Thr His Glu Gly Arg Lys
            35                  40                  45
   Leu Asn Gln Asn Ser Phe Arg Lys Ile Val Ser Glu Leu Thr Gln Glu
        50                  55                  60
   Asp Lys Lys Thr Leu Ile Asp Glu Phe Asn Glu Gly Phe Glu Gly Val
    65                  70                  75                  80
   Tyr Arg Tyr Leu Glu Met Tyr Thr Asn Lys
                    85                  90
<210> 2
   <211> 90
   <212> PRT
   <213> Bacteriophage T4
<400> 2
   Met Gly Lys Asn Ile Asp Thr Val Arg Glu Ile Ile Thr Val Ala Ser
     1               5                  10                  15
   Ile Leu Ile Lys Phe Ser Arg Glu Asp Ile Val Glu Asn Arg Ala Asn
   20 25 30
   Phe Ile Ala Phe Leu Asn Glu Ile Gly Val Thr His Glu Gly Arg Lys
   35 40 45
   Leu Asn Gln Asn Ser Phe Arg Lys Ile Val Ser Glu Leu Thr Gln Glu
   50 55 60
   Asp Lys Lys Thr Leu Ile Asp Glu Phe Asn Glu Gly Phe Glu Gly Val
   65 70 75 80
   Tyr Arg Tyr Leu Glu Met Tyr Thr Asn Lys
   85 90
<210> 3
   <211> 27
   <212> DNA
   <213> Artificial Sequence
<220>
   <223> Description of Artificial Sequence: PCR primer
<400> 3
   atggaattca acccgcagtc acagctg 27
<210> 4
   <211> 27
   <212> DNA
   <213> Artificial Sequence
<220>
   <223> Description of Artificial Sequence: PCR primer
<400> 4
   tgagtcgact taatcgtcga ggaagct 27
<210> 5
   <211> 25
   <212> DNA
   <213> Artificial Sequence
<220>
   <223> Description of Artificial Sequence: PCR primer
<400> 5
   ggccatgggc aataaaaaca ttgat 25
<210> 6
   <211> 26
   <212> DNA
   <213> Artificial Sequence
<220>
   <223> Description of Artificial Sequence: PCR primer
<400> 6
   ggggatcctt atttgttcgt atacat 26
<210> 7
   <211> 30
   <212> DNA
   <213> Artificial Sequence
<220>
   <223> Description of Artificial Sequence: PCR primer
<400> 7
   gaaagatctc atatgtcccc tatactaggt 30
<210> 8
   <211> 29
   <212> DNA
   <213> Artificial Sequence
<220>
   <223> Description of Artificial Sequence: PCR primer
<400> 8
   ctaagctttt atttgttcgt atacatctc 29

## Description

### TECHNICAL FIELD

The present invention relates to a method of identifying a ligand, in particular an inhibitor, of a bacterial RNA polymerase sigma subunit.

### BACKGROUND ART

### Sigma subunits of RNA polymerase

Transcription of genes to the corresponding RNA molecules is a complex process which is catalyzed by DNA dependent RNA polymerase, and involves many different protein factors. In eubacteria, the core RNA polymerase is composed of α, β, and β' subunits in the ratio 2:1:1. To direct RNA polymerase to promoters of specific genes to be transcribed, bacteria produce a variety of proteins, known as sigma (σ) factors, which interact with RNA polymerase to form an active holoenzyme. The resulting complexes are able to recognize and attach to selected nucleotide sequences in promoters.

Physical measurements have shown that the sigma subunit induces conformational transition upon binding to core RNA polymerase. Binding of the sigma subunit to the core enzyme increases the binding constant of the core enzyme for DNA by several orders of magnitude (Chamberlin, M.J. (1974) Ann. Rev. Biochem. 43, 721-).

Bacterial sigma factors do not have any homology with eukaryotic transcription factors, and are consequently a potential target for antibacterial compounds.

Mutations in the sigma subunit, effecting its association and ability to confer DNA sequence specificity to the enzyme, are known to be lethal to the cell.

Characterization of sigma subunits, identified and sequenced from various organisms, allows them to be classified into three groups. The Group I sigma has also been referred to the sigma⁷⁰ class, or the "house keeping" sigma group (for a review see Lonetto et al. (1992) J. Bacteriol. 174, 3843-3849). Sigma subunits belonging to this group recognize similar promoter sequences in the cell. These properties are reflected in certain regions of the proteins which are highly conserved between species.

Another important feature of sigma-dependent transcription is that the sigma subunit dissociates from the core enzyme during elongation of mRNA. Consequently, molecules which stabilize the interaction of the sigma subunit with the core enzyme would also be capable of inactivating transcription.

### Mycobacterium tuberculosis

*Mycobacterium tuberculosis* is a major pulmonary pathogen which is characterized by its very slow growth rate. As a pathogen it gains access to the alveolar macrophages where it multiplies within the phagosome, finally lysing the cells and being disseminated through the blood stream, not only to other areas of the lung, but also to extrapulmonary tissues.

The pathogen thus multiplies in at least two entirely different environments, involving the utilization of different nutrients and a variety of possible host factors. A successful infection would thus involve the coordinated expression of new sets of genes, transcribed by RNA polymerases associating with different sigma factors. This opens the possibility of targeting not only the sigma⁷⁰ subunits of *M. tuberculosis,* but also other sigma subunits specific for the different stages of infection and dissemination.

The cloning and expression of *Mycobacterium tuberculosis sigA* and *sigB* genes are disclosed in the International Patent Application WO 96/38478 (Astra AB).

### Anti-sigma factors

Anti-sigma (Asi) proteins are known in the art. Lysates of bacteriophage T₂ (Khesin et al. (1972) Mol. Gen. Genet 119, 299) or phage T₄ (Bogdanova et al. (1970) Mol. Biol. 4, 435; Stevens, A. (1972) Proc. Natl. Acad. Sci. U.S.A. 69, 603) have been reported to inhibit transcription of bacterial genes.

It has been established that the T₄-dependent anti-sigma⁷⁰ activity is borne by a 10 kDa protein (Stevens, A. In: RNA Polymerase p. 617-627 (Eds. R. Losick & M. Chamberlin) Cold Spring Harbor Laboratory, Cold Spring Harbor, N.Y., 1976). A 10 kDa protein was shown to co-purify with RNA polymerase from T₄ infected *E. coli* cells and is detached together with sigma⁷⁰ from the core enzyme on phosphocellulose columns.

A gene called *asiA,* coding for the 10 kDa anti-sigma⁷⁰ factor of bacteriophage T₄, has been identified by Orsini et al. (1993) J. Bacteriol. 175, 85-93. The open reading frame encoded a 90 amino acid protein with the deduced sequence MNKNIDTVRE IITVASILIK FSREDIVENR ANFIAFLNEI GVTHEGRKLN QNSFRKIVSE LTQEDKKTLI DEFNEGFEGV YRYLEMYTNK (SEQ ID NO: 5). The *asiA*-encoded protein (hereafter also referred to as "AsiA") was overproduced in a phage T₇ expression system and partially purified. It showed a strong inhibitory activity towards sigma⁷⁰-directed transcription by *E. coli* RNA polymerase holoenzyme. The nucleotide sequence of gene *asiA* has been deposited in the GenBank data base under accession no. M99441.

Examples of proteins regulating the sigma subunit of RNA polymerase are known also from other systems. An example is the *S. typhimurium* flagellar regulation system, which is a complex system controlled by a set of over 50 genes grouped into 13 flagellar operons. Late operon expression is positively regulated by the *fliA* gene coding for the sigma factor FliA (Suzuki et al. (1978) J. Bacteriol. 133, 904; Suzuki et al. (1981) J. Bacteriol. 145, 1036). On the other hand, the late operons are negatively regulated by the *flgM* gene. A 7.8 kDa protein has been identified as the *flgM* gene product and purified (Ohnishi et al. (1992) Mol. Microbiol. 6, 3149-3157). This FlgM protein was identified as an anti-sigma factor since it was capable to bind the FliA protein and disturbed its ability to form a complex with the RNA polymerase core enzyme.

Similarly, in *B. subtilis* gene expression, the sigma^{F} factor has been shown to be regulated by a 14 kDa anti-sigma factor encoded by the *spoIIAB* gene (Duncan & Losick (1993) Proc. Natl. Acad. Sci. U.S.A. 90, 2325-2329), while the sigma^{B} factor is regulated by a 16 kDa anti-sigma factor encoded by the *rsbW* gene (Benson & Haldenwang (1993) Proc. Natl. Acad. Sci. U.S.A. 90, 2330-2334).

The nucleotide sequences of the genes *flgM, spoIIAB* and *rsbW* are available in the GenBank data base (Accession Nos.: FLGMST.PRO for *flgM,* SPOIIAB.PRO for *spoIIAB,* M34995.PRO for *rsbW*). The sequences do not show any gross similarity with the *asiA* sequence disclosed by Orsini et al.

WO 96/25170 discloses methods for treating diseases caused by bacterial pathogens by administering the T₄ AsiA protein, which protein is shown to inhibit the RNA polymerase activity of *Bacillus subtilis* and *Mycobacterium smegmatis.*

### DISCLOSURE OF THE INVENTION

In accordance with the present invention, there is provided a method of identifying a ligand of a bacterial sigma⁷⁰ subunit which comprises contacting the sigma⁷⁰ subunit or a portion thereof comprising the anti-sigma binding region, with a test compound and a GST-AsiA fusion protein produced in a yeast expression system and determining whether the test compound binds competitively with the anti-sigma⁷⁰ factor to the sigma⁷⁰ subunit or portion thereof.

Preferably, the method comprises:
(i) immobilizing the sigma⁷⁰ subunit or portion thereof on a matrix or solid support;
(ii) adding the test compound and the fusion protein;
(iii) adding a first antibody against the fusion protein;
(iv) adding a labeled second antibody against the first antibody; and
(v) determining the amount of second antibody bound to the (first antibody - fusion protein - sigma⁷⁰ subunit or portion thereof) complex formed on the matrix or solid support.

The invention provides an *in vitro* method or assay for screening peptide libraries or chemical libraries for compounds (ligands) which mimic the anti-sigma⁷⁰ factor and which thereby have the capability of inhibiting the interaction of core RNA polymerase and sigma⁷⁰ subunits. Such inhibitor compounds are potentially useful in the treatment of bacterial infections.

The capability of the identified ligands to inhibit the interaction between a bacterial core RNA polymerase and a sigma⁷⁰ subunit can be tested by known methods, in particular by a RNA polymerase assay, such as that disclosed in Orsini et al *(supra)*, wherein transcription of a DNA template, such as calf thymus DNA, T₄ DNA or poly(dA-dT), is measured by the incorporation of a labeled RNA precursor such as [5-³H]UTP

In the method or assay according to the invention, the sigma⁷⁰ subunit or portion thereof is preferably obtained from *Escherichia coli* (particularly the C-terminal 99 amino acids of the sigma⁷⁰ subunit containing the anti-sigma binding region) or *Salmonella typhimurium.*

In the fusion protein, the anti-sigma⁷⁰ factor of bacteriophage T₄ will preferably have an amino acid sequence as shown in SEQ ID NO: 1 or SEQ ID NO: 2. The fusion protein comprises glutathione-S-transferase (GST-AsiA) as the marker gene protein product.

### BRIEF DESCRIPTION OF THE DRAWINGS

Fig. 1: Map of plasmid vector pARC 8112.
Fig. 2: Map of plasmid vector pARC 8100.
Fig. 3: Map of plasmid vector pARC 8115.
Fig. 4: Map of plasmid vector pARC 8101.
Fig. 5: Map of plasmid vector pARC 8114.
Fig. 6: Map of plasmid vector pARC 8105.
Fig. 7: Map of plasmid vector pARC 8180.
Fig. 8: Activity of GST-AsiA protein on sigma⁷⁰-dependent transcription. Assay: 0.5 µg *E. coli* core polymerase, 2.5 µg sigma⁷⁰ protein, 1.0 µg T₄ DNA and NTPs. Varying concentrations of GST-Asi was preincubated with sigma protein before addition to the reaction mixture. ³H-UTP specific activity was 2800 - 3000 cpm/nmole.

### EXAMPLES

Throughout this description the terms "standard protocols" and "standard procedures", when used in the context of molecular cloning techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

### EXAMPLE 1: Cloning, expression and purification of E. coli sigma⁷⁰

PCR primers corresponding to the 5'- and 3'-ends of the coding sequences of *E. coli* sigma were designed with the 5'-sequence including a site for the restriction enzyme *Eco*RI and the 3'-sequence including a site for the restriction enzyme *Sal*I. The PCR amplified DNA fragment was restricted with the above mentioned restriction enzymes and cloned into the *Eco*RI*-Sal*I sites of the expression vector pTrc99a (Amann et al. (1988) Gene 69, 301), whereafter the ligation mix was transformed into *E. coli* DH5α. Transformants harboring the recombinant plasmid with the expected restriction profile were identified.

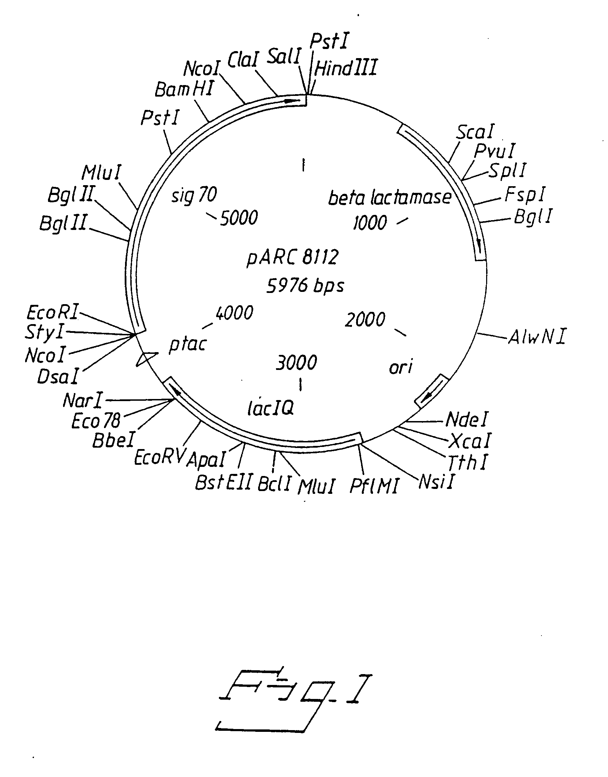

*E. coli* DH5α cells harboring the recombinant plasmid, labeled pARC 8112 (**Fig. 1**), were grown at +37°C in LB till an OD (600 nm) of 0.4 and induced with 1 mM IPTG. More than 50% of the 90 kDa sigma⁷⁰ protein was found as inclusion bodies as had been reported earlier (Borukhov & Goldfarb (1993) Protein expression and purification 4, 503). The overexpressed sigma⁷⁰ was purified and renatured following standard protocols.

The activity of the purified sigma⁷⁰ protein was confirmed by its ability to support transcription mediated by *E. coli* RNA polymerase core enzyme. *E. coli* RNA polymerase core and holoenzyme forms were purified following the protocol of Burgess and Jendriask (1975) Biochemistry 14, 4634-4638. Polymerase activity using T₄ DNA as template was assayed as described by Orsini et al. (1993) J. Bacteriol. 175, 85-93.

### EXAMPLE 2: Cloning, expression and purification of Salmonella typhimurium sigma⁷⁰

The coding sequence of the sigma⁷⁰ of *S. typhimurium* was amplified using the forward primer shown as SEQ ID NO: 3 and the reverse primer shown as SEQ ID NO: 4. The forward primer includes the site for the restriction enzyme *Eco*RI, while the reverse primer includes the sequence encoding the stop codon and a site for the restriction enzyme *Sal*I*. S. typhimurium* DNA was used as template and the amplified fragment digested with appropriate enzymes. This fragment was then ligated to *Eco*RI - *Sal*I digested pTrc 99A (Amann et al. (1988) Gene 69, 301) and transformed into *E. coli* DH5α to obtain the recombinant plasmid pARC 8118. Plasmid DNA of pARC 8118 was digested with *Eco*RI - *Hind*III and the released 2.3 kb DNA fragment encompassing the entire coding sequence of *S. typhimurium* sigma⁷⁰ was cloned into the *Eco*RI - *Hind*III digested pRSET B (Kroll et al. (1993) DNA and Cell Biol. 12, 441) vector which has not only the T₇ promoter but also sequences encoding a his tag which is now fused at the C-terminus of *S. typhimurium* sigma⁷⁰. The recombinant plasmid encoding the *S. typhimurium* sigma⁷⁰ fused to a His tag was labeled pARC 8133.

Plasmid DNA of pARC 8133 was then used to transform the expression host *E. coli* BL21(DE3) and transformants obtained at +37°C. Cells harboring pARC 8133 were grown in LB at +37°C till an OD of 0.4 at 600 nm and induced with 1 mM IPTG for 4 hours. The cells were pelleted, suspended in buffer and sonicated, according to standard methods. The lysed cells were clarified by centrifugation at 45,000 rpm for 60 min at +4°C and the clarified supernatant was loaded directly on Ni-agarose (Pharmacia) and washed with 50 mM imidazole. The bound protein was eluted with 200 mM imidazole and dialysed overnight at +4°C against 50 mM Tris-Cl, pH 7.5. The eluate was concentrated using Amicon cone filters to 1/10 volume. The concentrated protein was cleaved with enterokinase, used at a ratio of 1:50 of the enzyme, at +25°C for 14 hours. The cleaved mixture was passed through a Ni²⁺ agarose column and the unbound material collected. The cleaved protein was analyzed for homogeneity by SDS-PAGE.

### EXAMPLE 3: Toxicity of the anti-sigma protein

### 3.1. Cloning of the asiA gene

The coding sequence for the *asiA* gene (Orsini et al., *supra*) was amplified from the genomic DNA of bacteriophage T₄ by PCR using the forward primer shown as SEQ ID NO: 5 and the reverse primer designated shown as SEQ ID NO: 6.

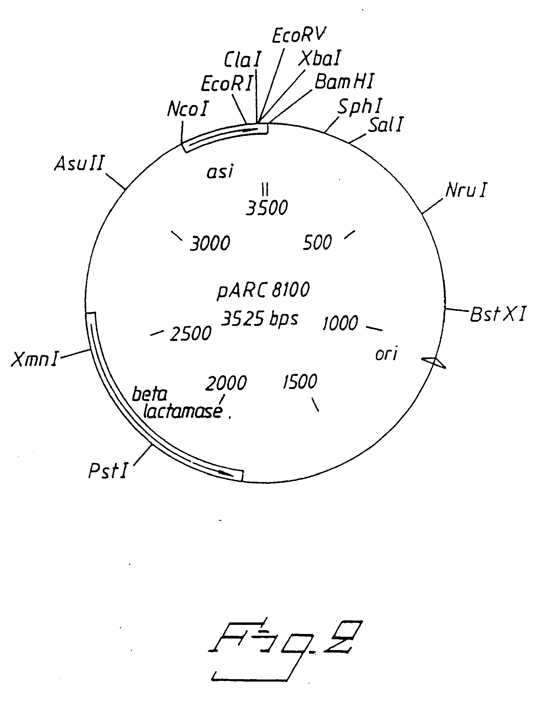

The 5'-primer included the sequence of the restriction enzyme *Nco*I while the 3'-primer included the sequence for the restriction enzyme *Bam*HI. The coding sequence was amplified by PCR following standard protocols and the amplified fragment ligated to *Nco*I *- Bam*HI restricted pBR329 and transformed into competent cells *E. coli* DH5α. Recombinants were selected at +37°C as chloramphenicol sensitive, ampicillin resistant transformants. One of the transformants having the desired restriction pattern was labeled pARC 8100 (**Fig. 2**).

The inclusion of the sequence encoding *Nco*I resulted in the change of the second amino acid from asparagine to glycine (SEQ ID NO: 2). The nucleotide sequence of the *asiA* gene cloned in pARC 8100 was verified by double stranded sequencing and found to be identical to the *asiA* sequence as disclosed by Orsini et al., except for the expected change of codon for the second amino acid as a result of the PCR cloning protocol used.

### 3.2. In vivo toxicity of asiA product in E. coli

### (A)

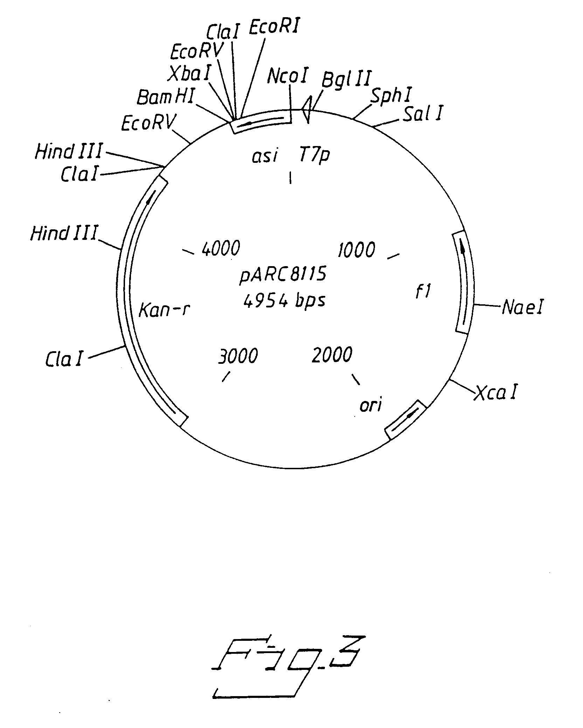

The *Nco*I *- Bam*HI DNA fragment from pARC 8100 was ligated to pET 8c Km (Umender K. Sharma et al., J. Bact., 177, 6745 (1996)) and kanamycin resistant transformants with *E. coli* DH5α were selected at +37°C. One of the transformants harboring a plasmid with the expected restriction enzyme profile was labeled pARC 8115 (**Fig. 3**). pARC 8115 plasmid DNA was then used to transform the expression host *E. coli* BL21(DE3) (Studier et al., *supra*) and transformants selected both at +37°C and at +30°C. However, no viable transformants could be obtained at either of the temperatures. The leaky expression from the T₇ promoter in pET 8c being much higher than from pET 11d(Km) (Studier et al., *supra*), the toxicity of the *asiA* product could explain the non-transformability.

Transformants could however be obtained using *E. coli* BL21(DE3)/pLysS as host, in which the leaky expression is additionally repressed by the T₇ lysozyme expressed from pLysS.

### (B)

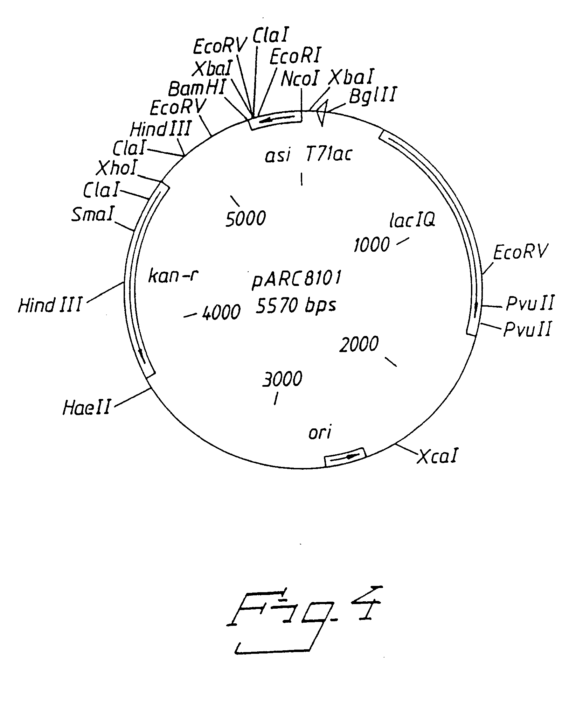

The *Nco*I *- Bam*HI 284 bp DNA fragment was obtained from pARC 8100 and ligated to the *Nco*I *- Bam*HI sites of pET 11d Km (Umender K. Sharma et al., J. Bact., 177, 6745 (1996)) and transformed into *E. coli* DH5α. Transformants were selected for kanamycin resistance. Plasmid preparations from individual transformants were digested with restriction enzymes and the correct transformant that released the 284 bp fragment after *Nco*I *- Bam*HI digest was labeled pARC 8101 (**Fig. 4**). The transformants were selected at +37°C and appeared normal.

pARC 8101 DNA was then used to transform the expression host *E. coli* BL26(DE3). BL26 is a lac Iq isogenic strain of BL21 (Studier et al., *supra*). Transformants selected for kanamycin both at +37°C and at +30°C.
The transformants obtained at +37°C were morphologically sick and non-viable indicating the acute toxicity of the leaky expression of the *asiA* gene. In contrast, healthy colonies could be obtained when transformants were selected at +30°C where the leaky expression from the T₇ promoter can be expected to be negligible.

BL26(DE3)/pLysS colonies were healthy both at +37°C and +30°C, indicating that the tight regulation of expression in this strain made the *asiA* gene non-toxic to the host. Transformants obtained from DH5α were also healthy both at +37°C and +30°C, indicating that the gene when transformed into a non-expression host was non-toxic.

### (C)

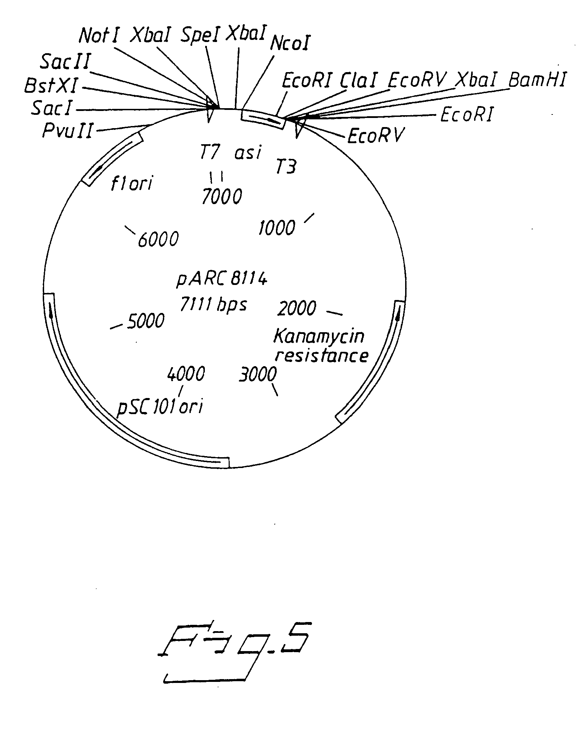

The *asiA* gene was excised from pARC 8101 as a *Xba*I *- Bam*HI DNA fragment to include the sequence for the ribosome binding site and ligated to the low copy vector, *Xba*I *- Bam*HI cleaved pWKS129 (Wang & Kushner (1991) Gene 100, 195) and the ligation mix transformed to *E. coli* DH5α. Recombinant plasmid harboring the *asiA* gene was identified by restriction profile and labeled pARC 8114 (**Fig. 5**).

This plasmid DNA when transformed into *E. coli* BL21(DE3) gave viable transformants both at +37°C and +30°C, since the low copy number of the plasmid reduced the level of the AsiA protein expressed through leaky expression.

### EXAMPLE 4: Glutathione-S-transferase - Anti-sigma fusion protein

### 4.1. Cloning of fusion protein in E. coli

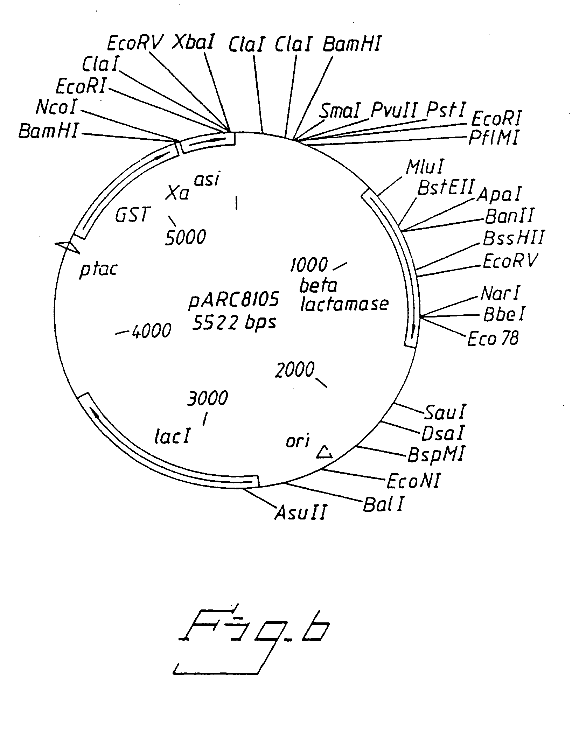

The coding sequence for the *asiA* gene was excised as an *Nco*I *- BamHI* fragment from pARC 8100 and ligated to *Nco*I *- BamHI* cleaved pARC 0499. The plasmid pARC 0499 has a *Nco*I site in frame with the glutathione-S-transferase encoding sequence, enabling fusion of the N-terminus of *asiA* sequences. The ligation mix was transformed into *E. coli* DH5α and transformants selected at +37°C and +30°C. Viable colonies were obtained at both temperatures indicating the N-terminal fusion reduces the toxicity of the protein. The recombinant plasmid obtained with the sequences encoding GST-AsiA was labeled pARC 8105 (**Fig. 6**).

The GST-AsiA fusion protein was purified as follows: *E. coli* DH5α harboring pARC 8105 was grown in LB till an OD at 600 nm of 0.6 at +37°C followed by the addition of 1 mM IPTG and allowing growth for a further period of 2 hours.
The cells were then centrifuged at 5000 rpm for 10 minutes. The pelleted cells were suspended in Buffer A (phosphate buffered saline (PBS) pH 7.5, 5 µg/ml aprotinin, 5 µg/ml leupeptin) and sonicated. The sonicate was clarified at 45,000 rpm for 10 minutes at +4°C and the supernatant passed through a Glutathione-Sepharose 4B column previously equilibrated with PBS. The column was washed with 5 bed volumes of 1 M urea once followed by washing with PBS (10 bed volumes). The bound protein was eluted with 10 mM glutathione and the eluate dialysed overnight against buffer containing 50 mM Tris(hydroxymethyl)aminomethane hydrochloride (Tris HCl), pH 7.5. The dialysed protein was then concentrated to 1/10 vol using Amicon cone filters. The concentrated protein was then treated with 1:50 ratio of Factor Xa protease in a buffer containing 50 mM Tris HCl, pH 7.5, 100 mM NaCl, 1 mM CaCl₂ at +25°C for 4 hours. The digested eluate was then passed through a Glutathione-Sepharose column and the unbound fraction collected and analyzed for the purity of the AsiA protein by SDS-PAGE.

### 4.2. Cloning of fusion protein in yeast

The expression and purification of the GST-AsiA fusion protein in *E*. *coli* did not allow large scale production because of the inherent toxicity of the fusion protein. To obtain large quantities of the AsiA protein, the possibility of expressing the GST-AsiA fusion protein in a yeast host was investigated. As the *Saccharomyces* species do not have a sigma⁷⁰ homologue, the AsiA protein was expected not to have toxicity effects on the transcription apparatus of the host and thus for initial experiments a *Saccharomyces cerevisiae* expression system was chosen.

The gene encoding the GST-AsiA fusion protein on the plasmid pARC 8105 was amplified using the forward primer shown as SEQ ID NO: 7 and the reverse primer shown as SEQ ID NO: 8. The amplified 1.0 kb fragment was restricted with *Bgl*II and *Hind*III which are the sites introduced by the primer sequence at the 5'-and 3'-ends of the amplified PCR fragment. The *Bgl*II - *Hind*III restricted fragment was ligated into the *Bgl*II- *Hind*III restricted *Saccharomyces cerevisiae* cloning vector pSW6 (Pascall et al. (1991) J. Mol. Endocrinol. 6, 63-70). The ligation mixture was transformed into the *Saccharomyces cerevisiae* host CGY1585 and transformants selected on leucine (3 µg/ml).

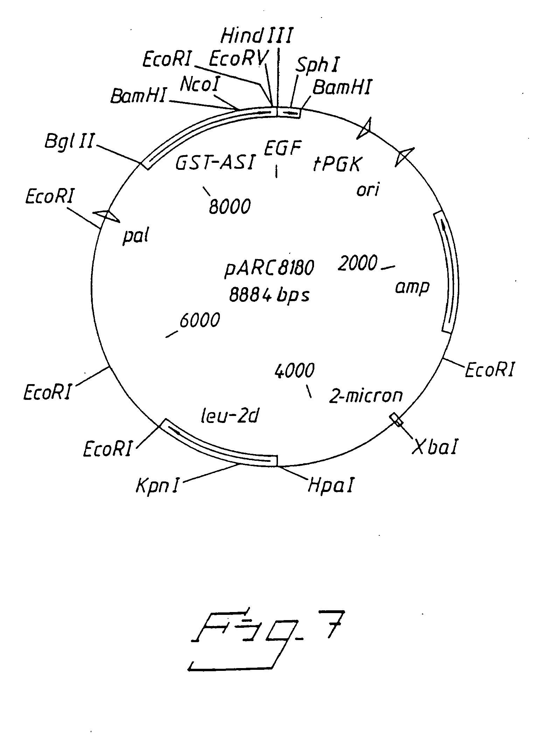

Individual transformants from both reaction mixtures were screened for the presence of plasmid with the expected restriction profile. The recombinant plasmid obtained with the vector PSW6 was labeled pARC 8180 (**Fig. 7**). *S. cerevisiae* CGY 1585 strain harbouring pARC 8180 were grown using standard procedures and lysed by passing the concentrated cell suspension through a French Press. The lysate was then centrifuged at 10,000 rpm and the supernatant applied to a Glutathione-Sepharose column (Pharmacia) and the fusion protein purified following standard protocols.

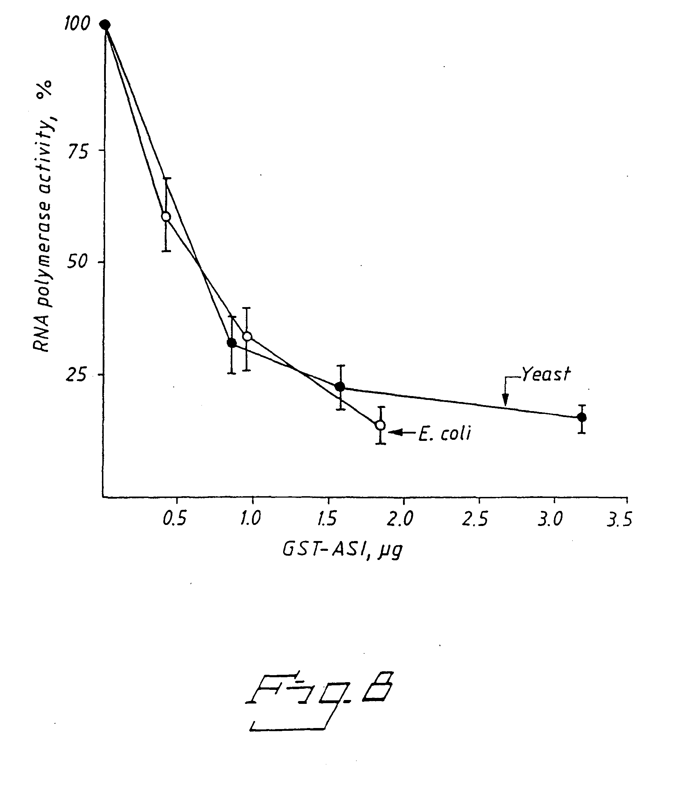

The purified GST-AsiA fusion protein was cleaved with Factor Xa as recommended and the AsiA protein separated. The yield of the purified AsiA protein was 500 µg/l. The activity of the GST-AsiA protein, expressed and purified from *Saccharomyces,* was compared to that obtained from *E. coli* and found to be identical in its ability to inhibit sigma⁷⁰ mediated transcription of T₄ template (**Fig. 8**). AsiA protein purified from GST-AsiA after Factor Xa cleavage also inhibited *E. coli* sigma⁷⁰ dependent transcription.

The ability to overexpress and purify large quantities of the AsiA protein in *Saccharomyces* without toxicity problems for the host also indicates that the AsiA protein does not have a corresponding sigma⁷⁰ homologue in this species. As the *Saccharomyces* RNA polymerase is similar to that of higher eukaryotes it also substantiates the fact that the AsiA protein is toxic only to the prokaryotic transcription apparatus.

In order to test the feasibility of expressing AsiA in alternative yeast expression systems, *Pichia pastoris* expression system was chosen. The gene encoding GST-AsiA was amplified by PCR from pARC 8105 and cloned into pPIC9K (Invitrogen).
5' primer (asi34) - TA TAC GTA TCC CCT ATA CTA GGT TAT TGG
3' primer (asi35) - T TGC GGC CGC TTA TTT GTT CGT ATA CAT

| | | |
|---|---|---|
| PCR conditions used were: | melting temperature | 94 °C |
| | annealing temperature | 50 °C |
| | extension temperature | 72 °C |

and 30 cycles were performed.

1Kb PCR product was cloned as SnaBI-NotI fragment into pPIC9K (pARC 8274). *Pichia pastoris* transformants of pARC 8274 were selected based on G 418 resistance. Expression studies indicated that GST-AsiA was secreted into the culture supernatant. GST-AsiA purified from the culture supernatant was found to inhibit *in vitro* transcription as efficiently as that of either *E. coli* or the *Saccharomyces* GST-AsiA.

This indicates that it is feasible to produce recombinant GST-AsiA in a yeast expression system.

### EXAMPLE 5: In vitro AsiA-sigma⁷⁰ interaction assays

### 5.1. RNA polymerase assay

The *E. coli* RNA polymerase assay was standardized following the protocol of Orsini et al. (J. Bacteriol. 175, 85-93, 1993) using T₄ phage DNA as template to quantify sigma⁷⁰ dependent transcription. *E. coli* RNA polymerase core enzyme was purified following the protocol of Burgess and Jendriask (1975) Biochemistry 14, 4634-4638.

### 5.2. Inhibition of E. coli transcription by AsiA protein

Increasing concentrations of AsiA protein expressed in *S. cerevisiae* was added to the reaction mixture and the transcription mediated by the *E. coli* RNA polymerase quantified. The results showed that increasing concentrations of AsiA protein completely inhibited transcription mediated by *E. coli* sigma⁷⁰.

### 5.3. Inhibition of E. coli transcription by GST-AsiA fusion protein

The GST-AsiA fusion protein was purified from *S. cerevisiae* as described in Section 5.2 and used at different concentrations in the *E*. *coli* sigma⁷⁰ mediated RNA polymerase transcription assay, using phage T₄ DNA as template. The GST-AsiA fusion protein inhibited >80% the sigma⁷⁰ mediated transcription when the core enzyme was reconstituted with 2.5 µg of sigma⁷⁰ protein.

### 5.4. Reactivation of E. coli sigma⁷⁰ mediated transcription

Increasing concentrations of *E. coli* sigma⁷⁰ protein were added to the reaction mixture containing 0.1 µg of AsiA. As shown in Table 1, addition of sigma⁷⁰ protein could reactivate transcription mediated by *E. coli* RNA polymerase. This reversal of inhibition demonstrates the specific interaction of the AsiA protein with sigma⁷⁰.

**TABLE 1**

| | *E. coli* sigma⁷⁰ | *S. typhimurium* sigma⁷⁰ |
|---|---|---|
| | [³H]UTP *(nmol)* | |
| *E. coli* RNA polymerase | 2.1 | 2.3 |
| *E. coli* RNA polymerase + 0.1 µg AsiA | 0.57 | 0.34 |
| *E. coli* RNA polymerase + 0.1 µg AsiA + 4 µg sigma⁷⁰ | 2.3 | 3.0 |
| *E*. *coli* RNA polymerase + 0.1 µg AsiA + 4 µg sigma⁷⁰ (AsiA preincubated) | 0.68 | 0.98 |

### 5.5. Effect of Salmonella typhimurium sigma⁷⁰ on E. coli transcription

As described in Section 5.1, the *E*. *coli* RNA polymerase activity assay was standardized with purified enzyme and phage T₄ DNA as template. The RNA polymerase could be >80% inhibited by 0.07 µg of purified AsiA protein.

To a reaction mixture containing the *E. coli* RNA polymerase, T₄ DNA and 0.1 µg of AsiA, increasing concentrations of purified *Salmonella typhimurium* sigma⁷⁰ was added and the RNA polymerase activity quantified. As shown in Table 1, addition of 4 µg of *S*. *typhimurium* sigma⁷⁰ could restore the activity of the RNA polymerase.

### 5.6. Preincubation of sigma⁷⁰ with AsiA

To a reaction mixture containing *E. coli* RNA polymerase and T₄ DNA template there was added 0.1 µg AsiA and 4 µg *E. coli* or *S. typhimurium* sigma⁷⁰, preincubated at +37°C for 10 min with 0.1 µg of AsiA. As shown in Table 1, preincubation of the sigma⁷⁰ with AsiA abolished the ability of the sigma⁷⁰, from both *E. coli* and *S. typhimurium,* to activate *E. coli* RNA polymerase.

### EXAMPLE 6: Competitive ELISA for quantification of AsiA-sigma⁷⁰ interaction

### Screening assays

The above principles provide a basis for a method of identification of oligonucleotide sequences encoding peptides, which may be either related or unrelated to the AsiA peptide, and which could efficiently bind to sigma⁷⁰ subunits from *E. coli, S. typhimurium* and/or other housekeeping or virulence-associated sigma subunits of bacterial pathogens.

The identification of such peptide structures would enable the further identification, by known methods, of peptoids, peptidomimetics and organic molecules which can be tested in transcription assays for inhibition of sigma⁷⁰ dependent transcription.

Truncated sigma⁷⁰ (C-terminal 99 amino acids) which has been shown to be the anti-sigma binding region of sigma⁷⁰ of *E*. *coli* (Severinova et al., J. Mol. Biol. (1996) 263 (5), pp 637-647) was cloned and expressed in a His tag expression vector resulting in a (His)₅-tagged protein product.

A solution of the protein product (200 pmoles/ml) in a pH 7.5 buffer comprising Tris HCl (10 mM) and sodium chloride (50 mM) was prepared and 200 µl of solution were added per well of a nickel-coated microtitre plate, followed by incubation at room temperature for a period of two hours. The microtitre plate was then washed 5 times with the buffer solution and then incubated at 37°C for one hour with a solution of bovine serum albumin (3% w/v) in the above buffer. After washing 5 times with phosphate buffered saline (PBS) with "Tween" 20 (polyoxyethylene (20) sorbitan monolaurate, 0.5% v/v), the GST-AsiA fusion protein prepared as described in *Example 4.1.* above (100 ng) was added together with a defined amount of a putative inhibitor of truncated sigma⁷⁰ protein (corallopyronin, myxopyronin or ripostatin) and incubated at 37°C for one hour. Further washing (5 times) with PBS-"Tween" 20 was carried out and then anti-GST antibodies (1:2000) raised in rabbits were added to the wells, with incubation at 37°C for one hour. The previous washing step was repeated and thereafter anti-rabbit IgG-horse radish peroxidase (HRP) conjugate (1:2000) was added and incubated at 37°C for one hour. A final wash with PBS-"Tween" 20 was carried out, before adding the HRP enzyme substrate, tetramethyl benzidene/ H₂O₂. The enzymic reaction (resulting in the development of colour) was stopped after a suitable period of time by the addition of 6N H₂SO₄ and the microtitre plate was "read" in a spectrophotometer using light of wavelength (λ) 450 nm. The results obtained are shown in Table 2 following.

**TABLE 2**

| Test | % Binding to truncated sigma⁷⁰ |
|---|---|
| GST-AsiA (500 ng/ml) | 100 |
| GST-AsiA + AsiA (7 µg/ml) | 12 |
| GST-AsiA + corallopyronin (30 µg/ml) | 69 |
| GST-AsiA + myxopyronin (30 µg/ml) | 56 |
| GST-AsiA + ripostatin (1 µg/ml) | 100 |
| GST-AsiA + ripostatin (10 µg/ml) | 55 |
| GST-AsiA + ripostatin (100 µg/ml) | 1.5 |

## Claims

1. A method of identifying a ligand of a bacterial sigma⁷⁰ subunit which comprises contacting the sigma⁷⁰ subunit or a portion thereof comprising the anti-sigma binding region, with a test compound and a GST-AsiA fusion protein produced in a yeast expression system, and determining whether the test compound binds competitively with the anti-sigma⁷⁰ factor to the sigma⁷⁰ subunit or portion thereof.

2. A method according to claim 1, which comprises:
(i) immobilizing the sigma⁷⁰ subunit or portion thereof on a matrix or solid support.
(ii) adding the test compound and the fusion protein;
(iii) adding a first antibody against the fusion protein;
(iv) adding a labeled second antibody against the first antibody; and
(v) determining the amount of second antibody bound to the (first antibody - fusion protein - sigma⁷⁰ subunit or portion thereof) complex formed on the matrix or solid support.

3. A method according to claim 1 or claim 2, wherein the sigma⁷⁰ subunit or portion thereof is obtained from *Escherichia coli* or *Salmonella typhimurium.*

4. A method according to any one of the preceding claims, wherein the AsiA protein has an amino acid sequence as shown in SEQ ID NO: 1 or SEQ ID NO: 2.

5. A method according to any one of the preceding claims wherein the ligand is an inhibitor of a bacterial sigma⁷⁰ subunit.

6. A method according to any one of the previous claims, wherein the fusion protein is produced in a Saccharomyces cerevisiae or Pichia pastoris expression system.

## Patentansprüche

1. Verfahren zur Identifizierung eines Liganden einer bakteriellen sigma⁷⁰-Untereinheit, wobei man in dem Verfahren die sigma⁷⁰-Untereinheit oder einen den Anti-sigma-Bindungsbereich umfassenden Teil davon mit einer Testverbindung sowie einem in einem Hefe-Expressionssystem produzierten GST-AsiA-Fusionsprotein in Kontakt bringt und bestimmt, ob die Testverbindung mit dem Anti-sigma⁷⁰-Faktor um die Bindung an die sigma⁷⁰-Untereinheit oder den Teil davon konkurriert.

2. Verfahren nach Anspruch 1, bei dem man:
(i) die sigma⁷⁰-Untereinheit oder den Teil davon an einer Matrix oder einem festen Träger immobilisiert,
(ii) die Testverbindung und das Fusionsprotein zugibt,
(iii) einen ersten Antikörper gegen das Fusionsprotein zugibt,
(iv) einen markierten zweiten Antikörper gegen den ersten Antikörper zugibt und
(v) die Menge an an dem auf der Matrix bzw. dem festen Träger gebildeten Komplex (erster Antikörper - Fusionsprotein - sigma⁷⁰-Untereinheit oder Teil davon) gebundenem zweiten Antikörper bestimmt.

3. Verfahren nach Anspruch 1 oder Anspruch 2, wobei die sigma⁷⁰-Untereinheit oder der Teil davon aus *Escherichia coli* oder *Salmonella typhimurium* gewonnen wird.

4. Verfahren nach einem der vorhergehenden Ansprüche, wobei das AsiA-Protein eine wie in SEQ ID NO: 1 oder SEQ ID NO: 2 dargestellte Aminosäuresequenz aufweist.

5. Verfahren nach einem der vorhergehenden Ansprüche, wobei es sich bei dem Liganden um einen Inhibitor einer bakteriellen sigma⁷⁰-Untereinheit handelt.

6. Verfahren nach einem der obigen Ansprüche, wobei das Fusionsprotein in einem Saccharomyces-cerevisiae- oder Pichia-pastoris-Expressionssystem produziert wird.

## Revendications

1. Procédé d'identification d'un ligand d'une sous-unité sigma⁷⁰ bactérienne qui consiste à mettre en contact la sous-unité sigma⁷⁰ ou une partie de celle-ci comprenant la région de liaison anti-sigma, avec un composé à l'essai et une protéine de fusion GST-AsiA produite dans un système d'expression chez la levure et à déterminer si le composé à l'essai entre en compétition avec le facteur anti-sigma⁷⁰ pour la liaison à la sous-unité sigma⁷⁰ ou à une partie de celle-ci.

2. Procédé selon la revendication 1, qui consiste à :
(i) immobiliser la sous-unité sigma⁷⁰ ou une partie de celle-ci sur une matrice ou un support solide ;
(ii) ajouter le composé à l'essai et la protéine de fusion ;
(iii) ajouter un premier anticorps dirigé contre la protéine de fusion ;
(iv) ajouter un deuxième anticorps marqué dirigé contre le premier anticorps ; et
(v) déterminer la quantité de deuxième anticorps lié au complexe (premier anticorps-protéine de fusion-sous-unité sigma⁷⁰ ou une partie de celle-ci) formé sur la matrice ou le support solide.

3. Procédé selon la revendication 1 ou la revendication 2, selon lequel la sous-unité sigma⁷⁰ ou une partie de celle-ci est obtenue à partir d'*Escherichia coli* ou de *Salmonella typhimurium.*

4. Procédé selon l'une quelconque des revendications précédentes, selon lequel la protéine AsiA possède une séquence d'acides aminés telle que représentée par SEQ ID NO : 1 ou SEQ ID NO : 2.

5. Procédé selon l'une quelconque des revendications précédentes, selon lequel le ligand est un inhibiteur d'une sous-unité sigma⁷⁰ bactérienne.

6. Procédé selon l'une quelconque des revendications précédentes, selon lequel la protéine de fusion est produite dans un système d'expression chez *Saccharomyces cerevisiae* ou *Pichia pastoris.*
